(19) Europäisches Patentamt
European Patent Office
Office européen des brevets

(11) Publication number: **0 351 625 B1**

(12) **EUROPEAN PATENT SPECIFICATION**

(45) Date of publication of patent specification: **07.04.93**

(51) Int. Cl.5: **A61K 31/71**, A61K 31/70

(21) Application number: **89112126.1**

(22) Date of filing: **03.07.89**

# (54) Anti-HIV activity of BV-3608.

(30) Priority: **01.07.88 US 214226**

(43) Date of publication of application:
**24.01.90 Bulletin 90/04**

(45) Publication of the grant of the patent:
**07.04.93 Bulletin 93/14**

(84) Designated Contracting States:
**AT BE CH DE ES FR GB GR IT LI LU NL SE**

(56) References cited:
**EP-A- 0 277 621**

**J. ANTIBIOTICS, vol. 41, no. 11, November 1988, pages 1708-1710 A. TANABE et al.: "Inhibitory effect of new antibiotic, pradimicin A on infectivity, cytopathic effect and replication of human immunodeficiency virus in Vitro"**

**J. ANTIBIOTICS, vol. 42, no. 2, February 1989, pages 344-346 H. HOSHINO et al.: "New antifungal antibiotics, benanomicins A and B inhibit infection of T-cell with human immunodeficiency virus (HIV) and syncytium formation by HIV"**

(73) Proprietor: **Bristol-Myers Squibb Company
345 Park Avenue
New York, N.Y. 10154(US)**

(72) Inventor: **Oki, Toshikazu
4-20-10 Shodo Totsuka-ku
Yokohama 247(JP)**
Inventor: **Yamamoto, Naoki
7-12, 1-chome Higashiobayamacho
Ube-shi Yamaguchi(JP)**

(74) Representative: **Kinzebach, Werner, Dr. et al
Patentanwälte Reitstötter, Kinzebach und Partner Sternwartstrasse 4 Postfach 86 06 49
W-8000 München 86 (DE)**

EP 0 351 625 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

J. ANTIBIOTICS, vol. 40, no. 3, March 1987, pages 396-399, H. NAKASHIMA et al.: "Inhibition by doxorubicin of human immunodeficiency virus (HIV) infection and replication In Vitro"

J. ANTIBIOTICS, vol. 41, no. 8, August 1988, pages 1019-1028 S. GOMI et al.: "The structures of new antifungal antibiotics, benanomicins A and B"

J. ANTIBIOTICS, vol. 41, no. 6, June 1988, pages 807-811, T. TAKEUCHI et al.: "New antifungal antibiotics, benanomicins A and B from an Actinomycete"

## Description

The invention relates to a method for inhibiting infection of a susceptible living biological in vitro substrate with HIV virus when said substrate is contacting said virus or living cellular material containing said virus which comprises providing a non-toxic antiviral effective amount of a BU-3608 compound in the presence of said substrate during said contacting.

The invention also relates to the use of a BU-3608 compound for preparing a medicament for treating or inhibiting HIV infection.

The BU- 3608 compounds have the the formula:

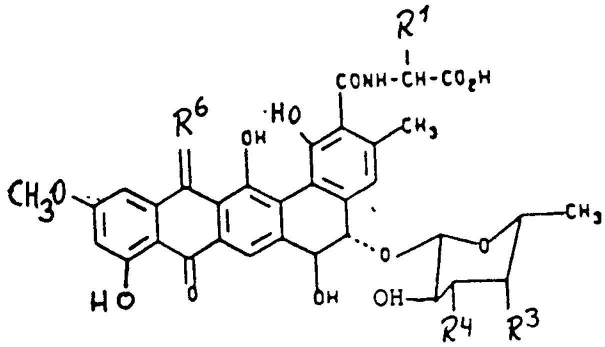

wherein $R^1$, $R^3$, $R^4$ and $R^6$ have the following meanings:

| Compound No. | $R^1$ | $R^3$ | $R^4$ | $R^6$ |
|---|---|---|---|---|
| 28567 | $-CH_3$(D) | $-NHCH_3$ | xylosyl | =O |
| 28747 | $-CH_3$(D) | $-NH_2$ | xylosyl | =O |
| 28786 | H | $-NHCH_3$ | xylosyl | =O |
| 28787 | H | $-NH_2$ | xylosyl | =O |
| 28634 | $-CH_3$ | $-NHCH_3$ | -H | =O |
| 28759 | $-CH_3$ | $-NHCH_3$ | xylosyl | =NH |

Human immunodeficiency virus (HIV) is a newly recognized retrovirus which is cytopathic for human T4 lymphocytes. This virus is believed to be the etiologic agent of the acquired immunodeficiency syndrome (AIDS) and AIDS-related complex (ARC). Although a number of antiviral agents; ribavirin, HPA-23, Suramin and phosphono formate, are now being tested for the therapy of AIDS, their clinical effects are not believed to be adequate. Recently, it has been proven that a nucleoside analog, 3-azido-3'-deoxythymidine (AZT) produced the prolongation of life span of AIDS patients and improved various symptoms accompanied with immunodeficiency, and thus AZT has been approved by governmental authorities for treating certain AIDS patients. It is considered that AZT functions through inhibition of reverse transcriptase which is the viral enzyme which converts viral RNA into DNA in infected cells. The selectivity of AZT is relatively high in vitro, and AZT suppresses the cellular damage and the expression of viral antigen by HIV infection. However, once the infection with HIV is established and provirus DNA is integrated into cellular DNA, AZT fails to inhibit virus production. AZT cannot inhibit the cell fusion of the virus producing cells and T4-positive cells by means of which cell to cell infection occurs.

The BU-3608 compounds which have been found capable of preventing the propagation of HIV in a growing cell system and are therefore believed to be useful in the treatment of AIDS and other conditions due to infection by this virus, are antifungal antibiotics which are produced as described in EP patent applications 88 101 410 (EP-A-277 621) and 89 110 234 (EP-A-345 735) filed February 02, 1988, and June 06, 1989 respectively. The structures of a number of representative BU-3608 compounds are illustrated in Table I hereof. Compound Nos. 28567, 28747, and 28634 are produced by fermentation with the microorganism Actinomadura hibisca Strain No. P157-2 and Strain No Q278-4, each of which have been deposited with the American Type Culture Collection under Accession Nos. ATCC 53557 and 53646. A third strain of this species, Strain No. P157-2-A-2660 is preferred for producing Compound Nos. 28786 and 28787 which can be obtained by fermentation of this microorganism which has been deposited with the American Type Culture Collection as ATCC No. 53762, according to the methods given in EP-A-277 621.

The preparation of quinonimine derivative of Compound No. 28567 is described herein as Compound No. 28759. The foregoing substances were previously known to possess antiviral activity against Herpes simplex virus Type I (HSVI) and influenza virus A but the finding of anti HIV activity as described herein was quite unexpected. A particularly surprising and significant aspect of the present invention is that the BU-3608 compounds while possessing strong anti HIV activity do not appear to have significant inhibitory action on the reverse transcriptase enzyme which is a common property of many of the prior anti HIV agents.

Figure 1 is a bar-graph in which the number of living cells is represented on the ordinate and the concentration of test compound in the culture medium is represented on the abscissa. Figure 1 refers to the effect of Compound No. 28567 on MT-4 cells grown in culture.

Figure 2 is a bar-graph in which the number of living cells is represented on the ordinate and the concentration of test compound is represented on the abscissa. This figure is similar to Figure 1 but refers to test results with Compound No. 28759.

Figure 3 also comprises a series of bar-graphs in which the ordinate represents a percentage value of cells grown in culture having the HIV specific antigen on their surfaces when infected cells are cultivated in the presence of various concentrations of test compound. The concentration of test compound is shown on the abscissa. Figure 3 represents results obtained with Compound No. 28567.

Figure 4 is similar to Figure 3 in that it is a series of bar-graphs in which the percentage of cells having the HIV specific antigen as represented in the immunofluorescence test (IF) versus concentration of test substance in the culture medium. The concentration is shown on the abscissa. Figure 4 represents results obtained with Compound No. 28759.

Figure 5 is made up of three graphs representing particle size distribution in which the number of particles is represented on the ordinate and the diameter of the particle on the abscissa. The particles involved are living MOLT-4 cells grown in culture and in each instance the proportion of particles exceeding 20 micrometers in diameter is shown by the shaded area under the curve and the percentage value is shown on the various graphs. Curves A and B shown in Figure 5 are pure cell cultures of the MOLT-4 cell line and the HTLV-IIIB infected MOLT-4 cell line. Curve C refers to a mixed culture containing equal numbers of the MOLT-4 cells and the MOLT-4/HTLV-IIIB cells.

Figure 6 are particle size distribution curves in which number of particles are plotted as ordinate and particle size as abscissa, each for a mixed culture of MOLT-4 and MOLT-4/HTLV-IIIB cells grown in the presence of either dextran Compound No. 28567, or Compound No. 28759 in the culture medium.

Since the BU-3608 compounds with which the present invention is concerned are believed to function by preventing cell-to-cell transfer of provirus DNA or of the virus per se, the BU-3608 compound may be administered to an AIDS patient at any stage of the disease where the immune system retains sufficient recuperative power to reestablish itself following elimination of the virus. Since the cells carrying the virus, or provirus DNA and are unable to infect fresh cells in the presence of the BU-3608 compound, the stage of the disease at which treatment is begun is not thought to be critical. Administration of the BU-3608 compound is for therapeutic or prophylactic treatment so long as cells bearing the virus or pro-virus DNA are present. The unique property makes the BU-3608 compounds suited for use according to the present invention in combination with other antiviral agents which are used to treat AIDS, particularly those such as AZT or other nucleoside analog which function by inhibition of reverse-transcriptase or other mechanism distinct from that operable with the BU-3608 compounds according to this invention.

The acute toxicity of Compound No. 28567 was determined in mice after single intravenous and intramuscular administrations. $LD_{50}$ after iv administration was 140 mg/kg; no sign of toxicity was observed after im dose of 400 mg/kg.

For treatment of HIV infections in animals and human beings, the BU-3608 compounds may be given in an antivirally effective amount by any accepted route of administration; these include, but are not limited, to, intravenous, intramuscular, oral, intranasal, and topical administration. Preparations for parenteral administration include sterile aqueous or non-aqueous solutions, suspensions or emulsions. They may also be manufactured in the form of sterile solid compositions which can be dissolved in sterile water, physiological saline, or some other sterile injectable medium immediately before use. Oral formulation may be in the form of tablets, gelatin capsules, powders, lozenges or syrups. For topical administration, the compound may be incorporated into lotions, ointments, gels, creams, salves or tinctures. Unit dosage forms may be prepared using methods generally known to those skilled in the art of pharmaceutical formulations.

It will be appreciated that when treating a host infected with HIV with a BU-3608 compound, the actual preferred route of administration and dosage used will be at the discretion of the attending clinician skilled in the treatment of such infections, and will vary according to the causative organism, its sensitivity to the antibiotic, severity and site of the infection, and patient characteristics such as age, body weight, rate of excretion, concurrent medications, and general physical condition. The skilled clinician will use as guidelines in determining the specific dose, the foregoing toxicity information, and the concentrations shown by the following cell culture experiments to lack cytotoxicity, and yet which possess the antiviral effect.

A cell culture system in which cells infected with the HTLV-III virus are treated with the test compound may be used to demonstrate the utility of the present substances. The cell line MT-4 disclosed by Harada, S. et al., in Virology, Vol. 146, pp. 272-281 (1985) is appropriate. This cell line features rapid and efficient replication of HTLV-III, followed by cytolysis. These characteristics facilitate detection of HTLV-IIIB, and provide a feasible system for demonstration of anti-AIDS utility in the laboratory.

MT-4 cells are maintained at 37°C in the complete medium. Two days before the start of an experiment, viable cells are counted by the trypan blue dye exclusion method and the concentration is adjusted to 30 x $10^4$ cells/mL.

Infection of the MT-4 cells with the virus is made at a multiplicity of infection (MOI) of 0.002. MT-4 cells (60 x $10^4$ cells/mL) are mixed with a 1:100 dilution of the viral preparation and incubated for 1 hour at 37°C for adsorption of the virus. After adsorption, HTLV-IIIB infected MT-4 cells are washed once and resuspended with complete medium.

MOLT-4/HTVL-IIIB cells are prepared at a concentration of 30 x $10^4$ cells/mL in RPMI 1640 medium supplemented with 10% decomplemented fetal calf serum (FCS), 100 IU/mL of penicillin, and 100 mcg mL of streptomycin (complete medium) and cultured for 4 days at 37°C. After centrifuging the cells at 1500 g for 10 minutes, the supernatant liquid of the MOLT-4/HTLV-IIIB cell culture is filtered through a 0.22 $\mu$m Millipore membrane to provide a viral preparation which is subdivided and stored in 1.0 mL portions at -80°C until use. The titer of this viral preparation is 6 x $10^5$ PFU/ml (PFU, plaque forming units).

The test substance is dissolved in dimethylsulfoxide (DMSO) and the solutions are then diluted with complete medium to concentrations of 10, 5.0, 2.5, and 1.25 mcg/ml to provide test solutions. Matched portions of HTLV-IIIB infected and uninfected MOLT-4 cells are adjusted to 60 x $10^4$ cells/mL and mixed with the same volume of diluted test solution to make the final concentration of 30 x $10^4$ cells/mL, and 5.0, 2.5, 1.125, and 0.63 mcg/mL of test substance. To measure anti-HIV activity, the number of viable cells surviving in the test medium are counted by the trypan blue dye-exclusion method on day 3 and day 6 after infection of the cells.

The expression rate of virus-specific antigen in the infected MT-4 cells, after incubation with media containing various concentrations of compounds, may be determined by an indirect immunofluorescence method (IF) on days 3 and 6 after infection. Cells are fixed with methanol at -20°C on a slide glass and incubated with 1:1,000-diluted anti-HIV positive human serum (IF titer, 1:4,096) for 30 min. at 37°C. The preparations are washed for 15 min. with phosphate-buffered saline, and the cells are then incubated with fluorescein isothiocyanate-conjugated rabbit anti-human immunoglobulin G (Dakapatts A/S, Copenhagen, Denmark) for 30 min. at 37°C, and washed again with phosphate-buffered saline. A sample of at least 500 cells is counted under a fluorescence microscope, and the percentage of IF-positive cells is calculated.

Staining of HTLV-III-specific antigens is performed substantially as described by Popovic, M., et al., Science, Vol. 224, pp. 497-500 (1984). On days 3 and 6 after infection, HTLV-IIIB infected MT-4 cells are smeared, dried and fixed with cold methanol for 3 minutes. Fixed cells are then incubated with 1:1000 diluted anti-HTLV-III positive human serum (IF titer; 1:4096) for 30 minutes at 37°C. The preparation is then washed for 15 minutes with phosphate buffered saline (PBS).

The fluorescein-isothiocyanate conjugated anti-human IgG (Dakopatts A/S, Copenhagen, Denmark) is applied, incubated for 30 minutes at 37°C and washed again with PBS. The cells are examined under a fluorescent microscope and the percent of IF-positive cells calculated from a sample of at least 500 cells.

The effect on cell fusion which results in cell to cell infection and giant cell formation may be examined as follows. MOLT-4 and its virus-producing cell, MOLT-4/HTLV-IIIB are mixed in 1:1 in proportion of cell number and adjusted to a final concentration of 5 x $10^5$ cells/ml with the medium containing various concentrations of compounds, and then incubated for 10 hours in a $CO_2$ incubator. After incubation, the distribution of cell size is measured by a cell multisizer (Coulter Electronics Ltd., Luton, England). Viable cells are counted by trypan blue dye exclusion method. Giant cell formation is observed under a phase-contrast microscope. Particles with diameters of from 3 to 100 $\mu$m in diameter are observed in the co-cultures. Only those particles having a diameter of more than 9 $\mu$m are considered to be cells. Cell sizes in each of the cultures of MOLT-4 or MOLT-4/HTLV-IIIB are similar, and the proportion of cell particles with 12-14 $\mu$m diameters are virtually the same in each culture. The percentage of cell particles having more than a 20 $\mu$m diameter was 2.7% and in the MOLT-4 cell culture, and 2.4% in the MOLT-4/HTLV-IIIB cell culture.

Cell particles with more than 50 $\mu$m in diameter were observed in co-cultures, and the proportion of cell particles having more than 20 $\mu$m diameter was 11.8%. This is a reflection of the infectivity by cell-to-cell fusion of the MOLT-4/HTLV-IIIB cells for the uninfected MOLT-4 cells. The test method measures the ability

of test substances to prevent or reduce this effect.

The utility of the following BU-3608 compounds shown by structure in Table I according to the present invention may be demonstrated by the foregoing methods.

Table I Representative BU-3608 Compounds

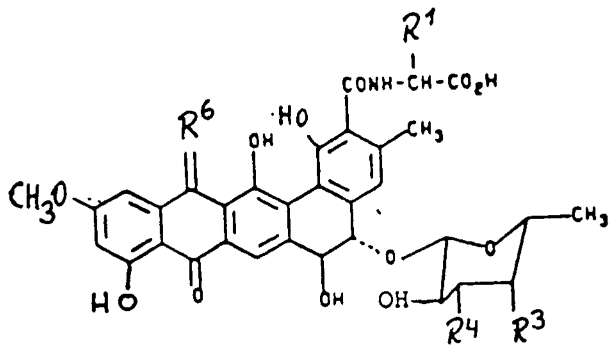

| Compound No. | Source | $R^1$ | $R^3$ | $R^4$ | $R^6$ |
|---|---|---|---|---|---|
| 28567 | 1 | $-CH_3$(D) | $-NHCH_3$ | xylosyl | =O |
| 28747 | 1 | $-CH_3$(D) | $-NH_2$ | xylosyl | =O |
| 28786 | 2 | H | $-NHCH_3$ | xylosyl | =O |
| 28787 | 2 | H | $-NH_2$ | xylosyl | =O |
| 28634 | 1 | $-CH_3$ | $-NHCH_3$ | -H | =O |
| 28759 | 3 | $-CH_3$ | $-NHCH_3$ | xylosyl | =NH |

1. EP-A-88 101 410
2. Recovered from the fermentation broth of Actinomadura hibisca P157-2-A-2660 (ATTC No. 53762) by methods described in EP-A-88 101 410
3. Preparative procedure given below.

Preparative Procedure for Compound No. 28759.

The hydrochloride salt of Compound No. 28567 (500 mg) was dissolved in 8N methanolic ammonia (200 mL) in an ice bath and the solution was stireed at 4° C for 65 hours. After concentration of the solution, the residue was dissolved in $H_2O$ (200 ml), adjusted to pH 3.0 and loaded on a column of Diaion HP-20 (300 ml). The column was washed with water and eluted with 80% aqueous acetone (pH 3.0). Evaporation of the purple colored eluate yielded the desired compound as the hydrochloride salt. An aqueous solution of

the hydrochloride (20 mg) was adjusted to pH 7.0 deposit the zwitterionic form of the desired quinonimine (6 mg), mp 150-155°C (dec.); IR(KBr) $cm^{-1}$ 3200, 1600, 1565, 1470, 1275;

$$UV\ \lambda_{max}^{50\%MeOH}$$

nm (ε) 239(21,200), 284(20,500), 330(8,900), 404(3,700), 542(9,500); SI-MS m/z 842 $(M+3H)^+$; Molecular formula $C_{40}H_{45}N_3O_{17}$; HPLC (System 1; column: YMC A-301-3 Yamamura Chem. Co., elution: $CH_3CN$/0.15% $KH_2PO_4$, pH 3.5, gradient: 0-3 min (20/80 - 35/65), 3-11 min (35/65), 11-16 min (35/65 - 55/45), Rt: 7.08 min.

Figure 1 illustrates the inhibition of cytopathic effect of the HIV virus on MT-4 cells by Compound No. 28567. First, the unshaded bars show the lack of cytotoxicity of the compound on uninfected MT-4 cells. Only at concentrations of 28567 in excess of 30 µg/ml is a noticeable or consistent reduction in the number of cells observed after 3 or 6 days, and even at 120 mg/ml, 60-70% of the cells survive.

The shaded bars of Figure 1 refer to the numbers of HIV-infected cells surviving after 3 or 6 days in the presence of various concentrations of Compound No. 28567 in the test medium. The zero concentration values reflect the lethality of the virus for the cells in this test system. Virtually complete protection is afforded the MT-4 cells at concentrations of Compound No. 28567 of 3.5 µg/ml and above.

Figure 2 refers to the evaluation of Compound No. 28759 in the same test system using MT-4 cells. Similar results were obtained with 28759 as were obtained with 28567.

Figures 3 and 4 refer to the percentage of MT-4 cells which express the HIV antigen when grown in culture for 3 or 6 days. Again test substance concentrations in the medium are shown as abscissa. All of the cells (100%) become positive within 6 days in the absence of any protective test drug. Compound Nos. 28567 and 28759 reduced the number of viral antigen positive cells to less than 1% of the population at concentrations of 3.5 µg/ml and greater.

Figure 5 is a group of three particle size distribution curves in which number of particles is shown as ordinate relative to particle diameter in µm as abscissa. A portion of the area under each curve is shaded to represent the proportion of particles having a diameter of 20 µm or greater. The shaded portion represents giant cells, fused cells, or syncytia. Curve A depicts the size distribution profile of the cells in a MOLT-4 cell culture, 2.7% of the cells exceed 20 µm in diameter. Curve B represents the size distribution profile of a MOLT-4/HTLV-IIIB culture, 2.4% of the cells exceed 20µm in diameter. Curve C refers to mixed culture of equal parts of MOLT-4 cells and MOLT-4/HTLV-IIIB cells, 11.8% of the cells exceeding 20 µm in diameter signifying the cell-to-cell infection process through cell fusion.

Figure 6 is a group of three particle size distribution curves for mixed cultures of MOLT-4 and MOLT-4/HTLV-IIIB similar to Curve C of Figure 5 except that for Curve D, 50 µg/ml of dextran was added to the medium; for Curve E, 25 µg/ml of Compound No. 28567 was added to the medium; and for Curve F, 25 µg/ml of Compound No. 28759 was added to the medium. In each instance the 11.8% value for proportion of particles exceeding 20 µm (Curve C of Figure 5) was reduced respectively to 1.9% with dextran sulfate (Curve D), 2.7% with Compound No. 28567 (Curve E), and 4.4% with Compound No. 28759 (Curve F). These results demonstrate the ability of the test compounds to prevent cell-fusion of MOLT-4 cells by the HTLV-IIIB virus.

## Claims

**1.** The use of a BU-3608 compound of the formula:

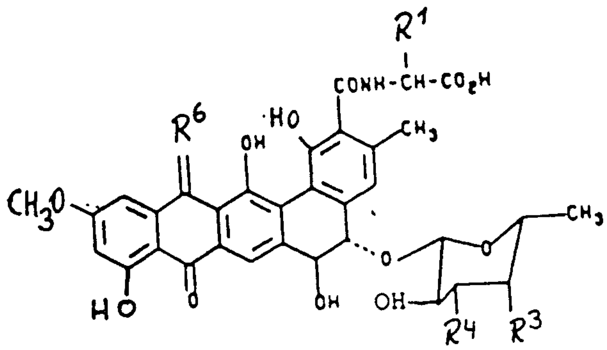

wherein $R^1$, $R^3$, $R^4$ and $R^6$ have the following meanings:

| $R^1$ | $R^3$ | $R^4$ | $R^6$ |
|---|---|---|---|
| $-CH_3$(D) | $-NHCH_3$ | xylosyl | =O |
| $-CH_3$(D) | $-NH_2$ | xylosyl | =O |
| H | $-NHCH_3$ | xylosyl | =O |
| H | $-NH_2$ | xylosyl | =O |
| $-CH_3$ | $-NHCH_3$ | -H | =O |
| $-CH_3$ | $-NHCH_3$ | xylosyl | =NH |

for preparing a medicament for inhibiting or treating infection of a susceptible living biological substrate with HIV virus.

**2.** The use of Claim 1 wherein said biological substrate is a cell culture growing in vitro.

**3.** The use of Claim 1 wherein said biological substrate is a cell culture of MT-4 cells growing in vitro.

**4.** The use of Claim 1 wherein said susceptible substrate is within a host animal infected with HIV.

**5.** The use of Claim 1 wherein said susceptible substrate is within a human subject infected with HIV.

**6.** A method for inhibiting infection of a susceptible living biological in vitro substrate with HIV virus when said substrate is contacting said virus or living cellular material containing said virus which comprises providing a non-toxic antiviral effective amount of a BU-3608 compound as defined in claim 1 in the presence of said substrate during said contacting.

**7.** The method of claim 6 wherein said biological substrate is a cell culture growing in vitro.

**8.** The method of claim 6 wherein said biological substrate is a cell culture of MT-4 cells growing in vitro.

## Patentansprüche

**1.** Verwendung einer BU-3608-Verbindung der Formel:

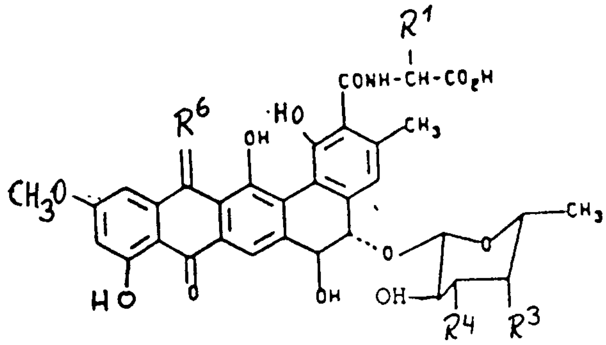

worin $R^1$, $R^3$, $R^4$ und $R^6$ die folgenden Bedeutungen besitzen:

| $R^1$ | $R^3$ | $R^4$ | $R^6$ |
|---|---|---|---|
| $-CH_3$(D) | $-NHCH_3$ | Xylosyl | =O |
| $-CH_3$(D) | $-NH_2$ | Xylosyl | =O |
| H | $-NHCH_3$ | Xylosyl | =O |
| H | $-NH_2$ | Xylosyl | =O |
| $-CH_3$ | $-NHCH_3$ | -H | =O |
| $-CH_3$ | $-NHCH_3$ | Xylosyl | =NH |

zur Herstellung eines Arzneimittels zur Inhibierung oder Behandlung der Infektion eines suszeptiblen lebenden biologischen Substrates mit dem HIV-Virus.

**2.** Verwendung nach Anspruch 1, wobei das biologische Substrat eine in vitro-wachsende Zellkultur ist.

**3.** Verwendung nach Anspruch 1, wobei das biologische Substrat eine in vitro-wachsende Zellkultur von MT-4-Zellen ist.

**4.** Verwendung nach Anspruch 1, wobei das suszeptible Substrat sich in einem mit HIV infizierten tierischen Wirt befindet.

**5.** Verwendung nach Anspruch 1, wobei das suszeptible Substrat sich in einem mit HIV-infizierten Menschen befindet.

**6.** Verfahren zur Inhibierung einer Infektion eines suszeptiblen lebenden biologischen in vitro-Substrates mit dem HIV-Virus, wenn das Substrat mit dem Virus oder lebendem, das Virus enthaltendem Zellmaterial in Kontakt kommt, wobei man eine nicht-toxische antiviral wirksame Menge einer BU-3608-Verbindung nach Anspruch 1 in Anwesenheit des Substrates während der Kontaktzeit bereitstellt.

**7.** Verfahren nach Anspruch 6, wobei das biologische Substrat eine in vitro-wachsende Zellkultur ist.

**8.** Verfahren nach Anspruch 6, wobei das biologische Substrat eine in vitro-wachsende Zellkultur von MT-4-Zellen ist.

## Revendications

**1.** Utilisation d'un composé de BU-3608 de la formule :

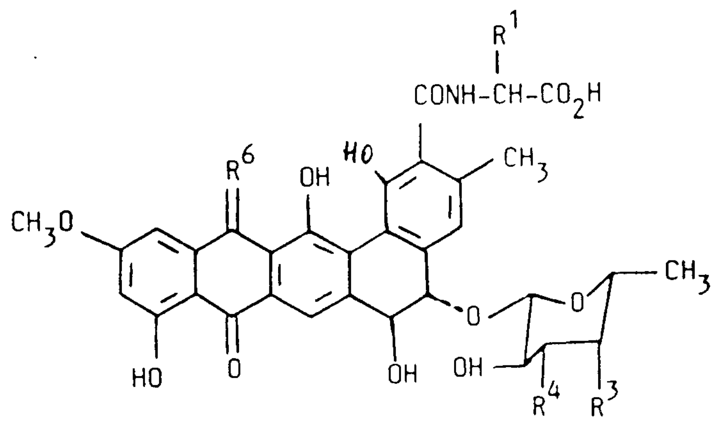

où $R^1$, $R^3$, $R^4$ et $R^6$ ont les significations suivantes :

| $R^1$ | $R^3$ | $R^4$ | $R^6$ |
|---|---|---|---|
| $-CH_3$(D) | $-NHCH_3$ | xylosyle | =O |
| $-CH_3$(D) | $-NH_2$ | xylosyle | =O |
| H | $-NHCH_3$ | xylosyle | =O |
| H | $-NH_2$ | xylosyle | =O |
| $-CH_3$ | $-NHCH_3$ | -H | =O |
| $-CH_3$ | $-NHCH_3$ | xylosyle | =NH |

pour la préparation d'un médicament pour l'inhibition ou le traitement d'une infection d'un substrat sensible biologique vivant par le virus HIV.

**2.** Utilisation de la revendication 1, où ledit substrat biologique est une culture de cellules croissant in vitro.

**3.** Utilisation de la revendication 1, où ledit substrat biologique est une culture de cellules de cellules MT-4 croissant in vitro.

**4.** Utilisation de la revendication 1, où ledit substrat sensible est dans un hôte animal infecté par HIV.

**5.** Utilisation de la revendication 1, où ledit substrat sensible est dans un sujet humain infecté par HIV.

**6.** Méthode pour l'inhibition de l'infection d'un substrat sensible biologique vivant in vitro par le virus HIV quand ledit substrat est mis en contact avec ledit virus ou un matériel cellulaire vivant contenant ledit virus, qui consiste à prévoir une quantité non toxique antivirale efficace d'un composé de BU-3608 tel que défini à la revendication 1 en présence dudit substrat pendant ladite mise en contact.

**7.** Méthode de la revendication 6, où ledit substrat biologique est une culture de cellules croissant in vitro.

**8.** Méthode de la revendication 6, où ledit substrat biologique est une culture de cellules MT-4 croissant in vitro.

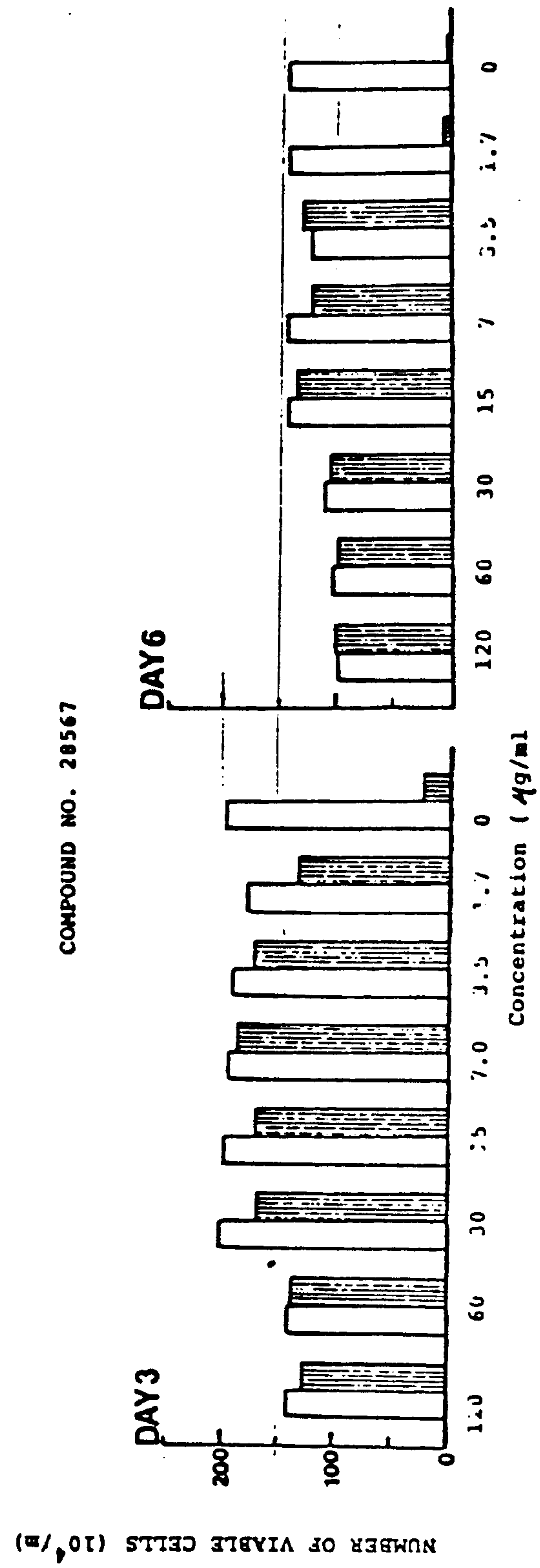
COMPOUND NO. 28567
DAY3
DAY6
NUMBER OF VIABLE CELLS ($10^4$/m)
0
100
200
Concentration ( μg/ml
120
60
30
15
7
0

FIGURE 1

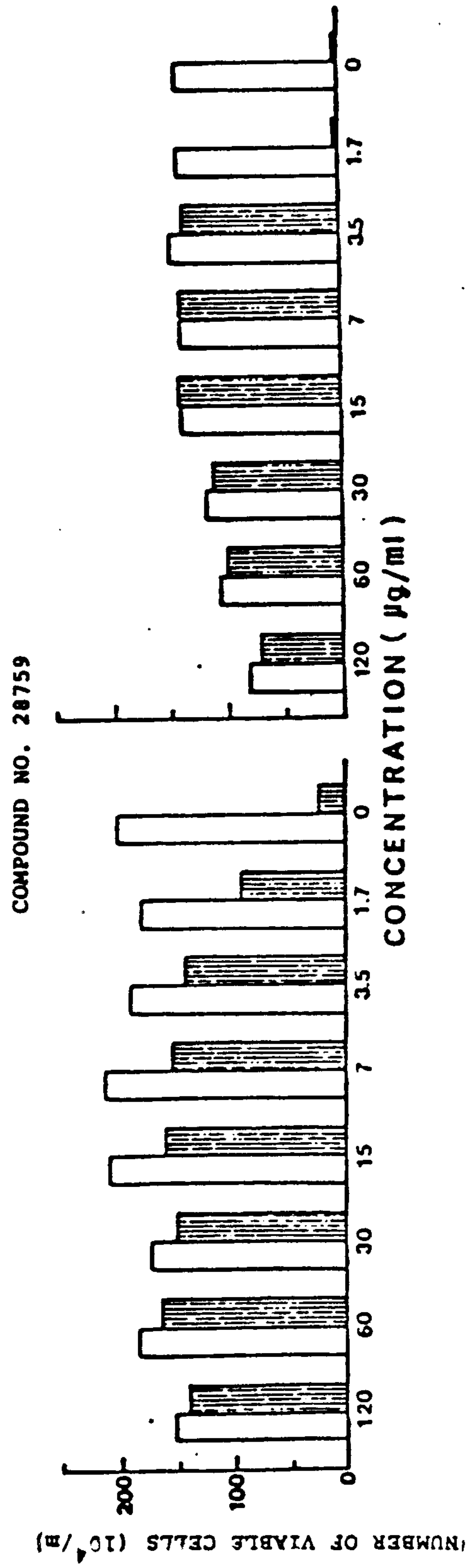
COMPOUND NO. 28759
NUMBER OF VIABLE CELLS (10⁴/ml)
200
100
0
120
60
30
15
7
3.5
1.7
0
CONCENTRATION ( μg/ml )

FIGURE 2

COMPOUND NO. 28567

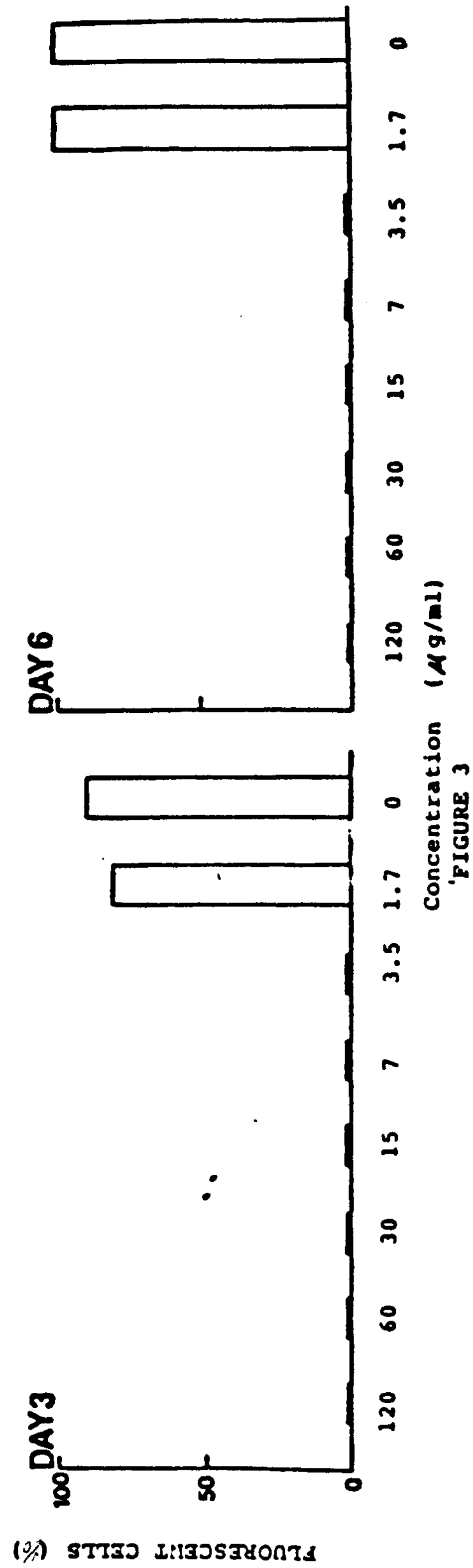


FIGURE 3

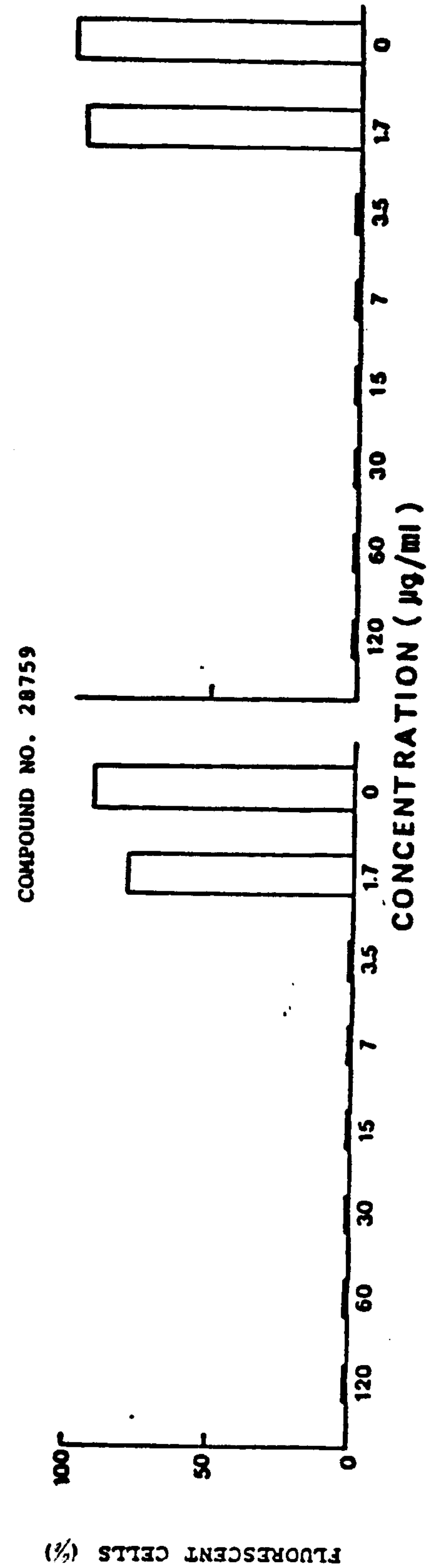
COMPOUND NO. 28759
FLUORESCENT CELLS (%)
100
50
0
120
60
30
15
7
3.5
1.7
0
120
60
30
15
7
3.5
1.7
0
CONCENTRATION ( μg/ml )

FIGURE 4

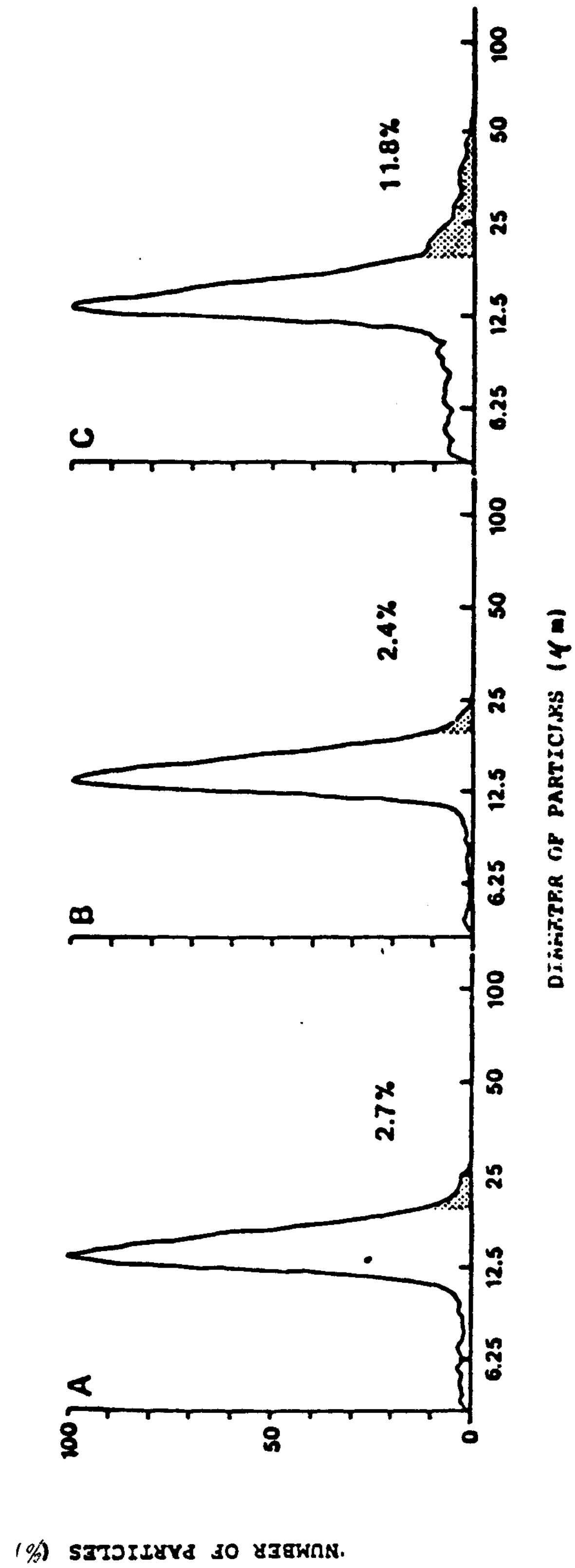
A
B
C
2.7%
2.4%
11.8%
100
50
0
6.25
12.5
25
50
100
DIAMETER OF PARTICLES (μm)
NUMBER OF PARTICLES (%)

FIGURE 5

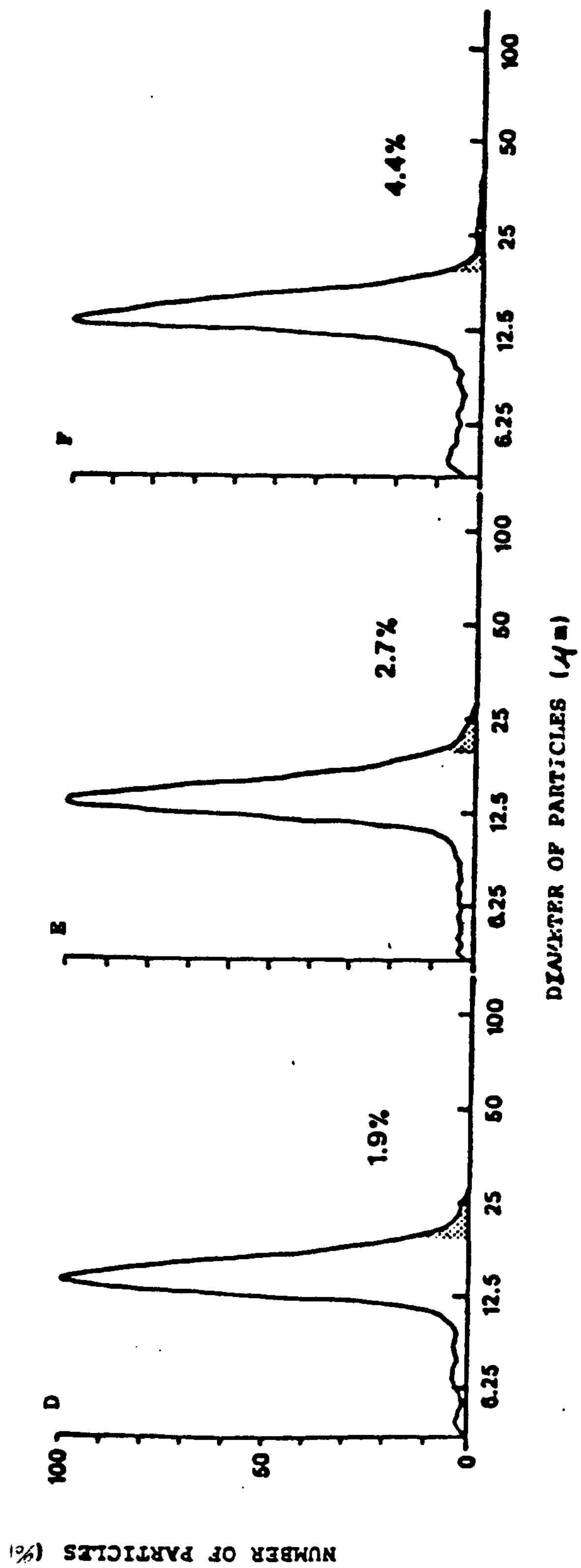
D
E
F
1.9%
2.7%
4.4%
0
50
100
6.25
12.5
25
50
100
DIAMETER OF PARTICLES (μm)
NUMBER OF PARTICLES (%)

FIGURE 6